# EUROPEAN PATENT SPECIFICATION

(11) **EP 4 676 344 B1**
(45) Date of publication and mention of the grant of the patent: **25.11.2026**
(21) Application number: 24707556.7
(22) Date of filing: 01.03.2024
(51) Int. Cl.: A61B 17/04, A61B 17/11

(54) **IMPLANTABLE ANASTOMOSIS DEVICE**
IMPLANTIERBARE ANASTOMOSEVORRICHTUNG
DISPOSITIF D'ANASTOMOSE IMPLANTABLE

(30) Priority: 09.03.2023 EP 23161077
(43) Date of publication of application: 14.01.2026
(73) Proprietor: UNIVERSITE DE GENEVE, 1211 Geneva 4 (CH)
(72) Inventor: OLDANI, Graziano, 1206 Genève (CH)
(74) Representative: reuteler & cie SA
(86) International application number: PCT/EP2024/055326
(87) International publication number: WO 2024/184211

(56) References cited:
- WO-A1-2022/171349
- US-A1- 2015 320 424
- US-A1- 2016 074 041

## Description

The present invention relates to an implantable anastomosis device, particularly for a pancreaticoduodenectomy procedure. The present invention also relates to a tool kit accompanying the implantable anastomosis device to assist in the surgical procedure. Resection and anastomosis are typically performed with conventional surgical instruments without the use of an implantable device. In procedures with very soft tissues, such as in Pancreaticoduodenectomy, a significant drawback is the risk of dehiscence of the anastomosis interface after operation, and the associated complications arising from such rupture. It is also difficult to correctly align and connect the sectioned pancreatic tissue to the small intestine.

There is a desire to provide a device and tools that allow the surgeon to operate more accurately, reliably and in a repeatable manner, and further to decrease the risk of rupture of the connection between the organs.

An object of the invention is to provide an implantable anastomosis device, particularly for a pancreaticoduodenectomy procedure, that simplifies surgical procedures while increasing the quality, reliability and repeatability thereof as well as increasing the strength of the anastomosis interface and reducing the risk of post operative rupture.

It is advantageous to provide an implantable anastomosis device that is compact, economical and easy to manipulate during surgery.

It is advantageous to provide an implantable anastomosis device that is robust and easy to manipulate.

It is advantageous to provide an implantable anastomosis device that facilitates the suture of resected interface tissues.

US 2016/074041 A1 relates generally to the field of medical devices and, in particular, to devices and methods for reconnecting two hollow body parts, such as a urethra to a bladder. US 2015/320424 A1 relates to a compression anastomosis system, particularly a compression anastomosis system for performing an anastomosis of the colon by minimally invasive surgery. WO 2022/171349 A1 relates to a size-variable implantable device, and in particular, though not exclusively, to a self-expandable implantable device configured for securing the device to a surgical site of a patient.

Objects of the invention have been achieved by providing a system according to claim 1. Dependent claims set out various advantageous features of embodiments of the invention. Disclosed herein is an implantable anastomosis device for use in a surgical connection between two tissues, comprising a support ring having a central cavity configured for receiving a first section of tissue therein, a plurality of spikes integrally formed with or attached to the support ring and arranged to project into the central cavity, and a plurality of suture guide holes, or at least one peripheral tie groove, formed through or on the support ring and configured for securing a second section of tissue to the support ring with suture thread.

In an advantageous embodiment, at least one of a lower or an upper side of the central cavity of the support ring comprises a spike free central zone that does not comprise a spike.

In an advantageous embodiment, the support ring has an oval shape.

In an advantageous embodiment, the support ring is made of a non-absorbable polymer material, for instance nylon, or an absorbable polymer, for instance poly lactic-co-glycolic acid, and wherein the support ring is made by injection molding or 3D printing.

In an advantageous embodiment, the support ring comprises an upper portion and a lower portion movable with respect to each other from an open position configured to place the first section of tissue on one of the lower portion and upper portion, to a closed position in which the lower portion and upper portion form closed ring configured to fully surround the first section of tissue.

In an advantageous embodiment, the upper and lower portions prior to use are separate parts configured to be assembled together in use.

In an embodiment, the upper and lower portions prior to use are connected together via a pivot hinge.

In an advantageous embodiment, the support ring comprises said at least one tie groove formed in periphery of the support ring.

In an advantageous embodiment, the support ring comprises a plurality of tie grooves, one tie groove formed in a periphery of a central body portion and at least another tie groove formed in a rim on a front side or a back side of the central body portion.

In an advantageous embodiment, the support ring comprises said plurality of suture guide holes.

In an advantageous embodiment, the plurality of suture guide holes extending axially through an outer ridge of the support ring.

In an advantageous embodiment, the device is configured for Pancreaticoduodenectomy.

In an advantageous embodiment, the device is configured for termino-terminal pancreatico-jejunal anastomosis.

In an advantageous embodiment, the device is configured for termino-lateral pancreatico-jejunal anastomosis.

Also disclosed herein is a tool kit for assembly and manipulation of the anastomosis device configured for termino-terminal pancreatico-jejunal anastomosis, including a holder fork comprising a finger grip and a pair of spaced apart arms with catches configured for removably coupling to an outer portion of the support ring.

In an advantageous embodiment, the tool kit further includes any one or more of a suture alignment tool comprising suture guide tubes and a tie alignment tool, each comprising locating pin portions configured for engaging complementary positioning elements on the holder fork.

Further advantageous features of the invention will be apparent from the following detailed description of embodiments of the invention and the accompanying illustrations.

### Brief description of the figures

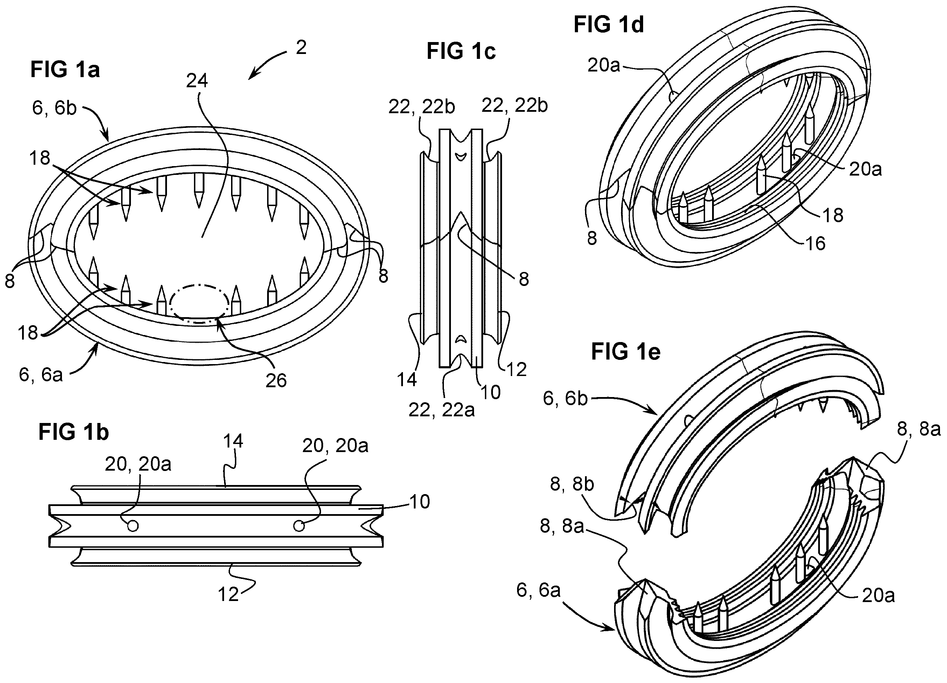

Figure 1a is a front view of an implantable anastomosis device according to an embodiment of the invention;
Figures 1 b and 1c are top and side views respectively of the device of figure 1a;
Figure 1d is a perspective view of the device of figure 1a;
Figure 1e is a perspective exploded view of the device of figure 1d;
Figure 2 is an exploded perspective view of a tool kit and an implantable anastomosis device of a medical anastomosis kit according to an embodiment of the invention;
Figures 3a to 3i are perspective views illustrating the assembly of an implantable anastomosis device to a section of pancreas and the connection of a section of the small intestine to the pancreas, using the tool kit according to an embodiment of the invention;
Figure 4a is perspective view of another embodiment of an implantable anastomosis device according to an embodiment of the invention;
Figures 4b and 4c are front and side views of the embodiment of figure 4a, shown in an open position prior to assembly around a section of pancreas;
Figures 5a to 5g are perspective views and figure 5h a cross-sectional longitudinal view showing the assembly of the embodiment of figures 4a to 4c to a section of pancreas and a section of the small intestine (termino-lateral pancreaticojejunostomy);
Figures 6a and 6b are front views of yet another embodiment of an implantable anastomosis device, 6a showing the position prior to coupling to a section of tissue and 6b in a coupled position.

Referring to the figures, a medical anastomosis kit comprises an implantable anastomosis device 2 and optionally a tool kit 3 for facilitating the manipulation and assembly of the implantable anastomosis device to the tissue sections it is intended to couple. The present invention is particularly suitable for coupling the pancreas to the small intestine, namely Pancreaticoduodenectomy.

The pancreas is a very soft tissue with low mechanical resistance that is difficult to securely fix to the intestinal wall, the medical device described herein is thus particularly well suited for Pancreaticoduodenectomy.

The implantable anastomosis device 2 comprises a support ring 6 having a central cavity 24 to receive a section of tissue therein (referred to herein as "first tissue section").

The implantable anastomosis device 2 may be made of various *per se* known certified implantable materials including non-absorbable polymers such as nylon or absorbable (biodegradable) polymers such as poly lactic-co-glycolic acid (PLGA). The implantable anastomosis device 2 may for instance be made by injection molding or 3D printing.

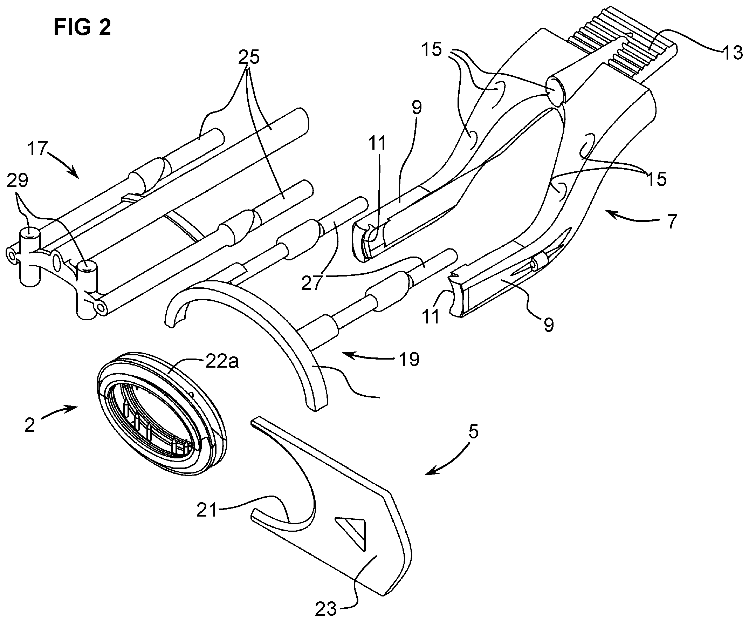

In the embodiments of figures 1a to 5h, the support ring comprises two portions that can be opened apart to be mounted around the first tissue section.

In the embodiment of figures 1a to 5h, the support ring comprises an upper ring portion 6a and a lower ring portion 6b that are separate parts, substantially ring halves, that have a coupling joint 8 with complementary guide and positioning profiles 8a, 8b that inter-engage to securely position the upper and lower ring portions together once in the assembled position as illustrated in figures 1a to 1d.

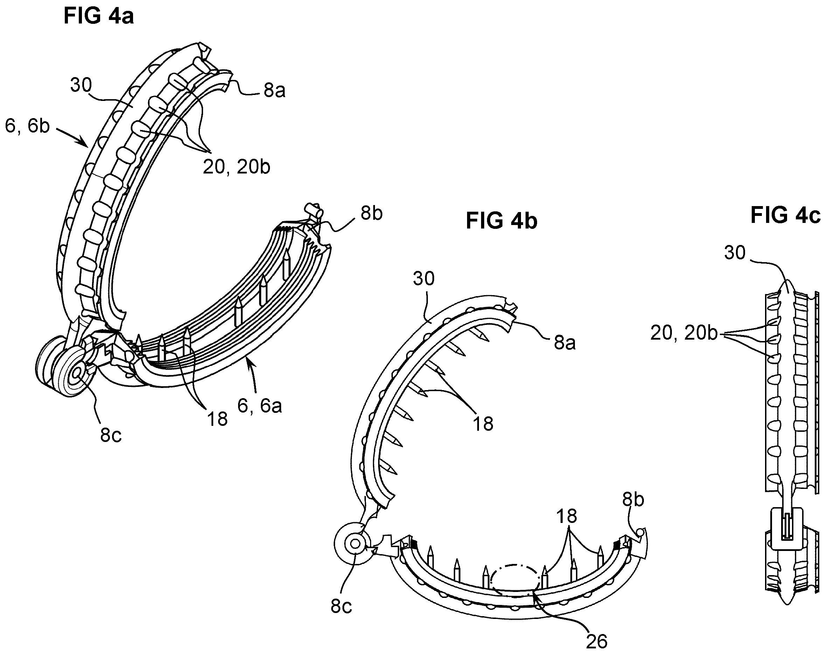

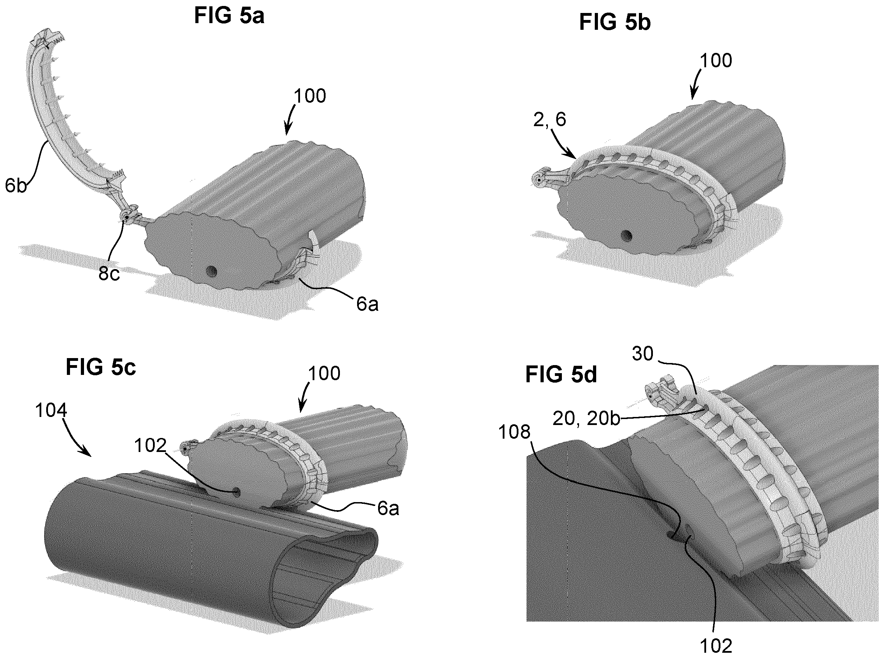

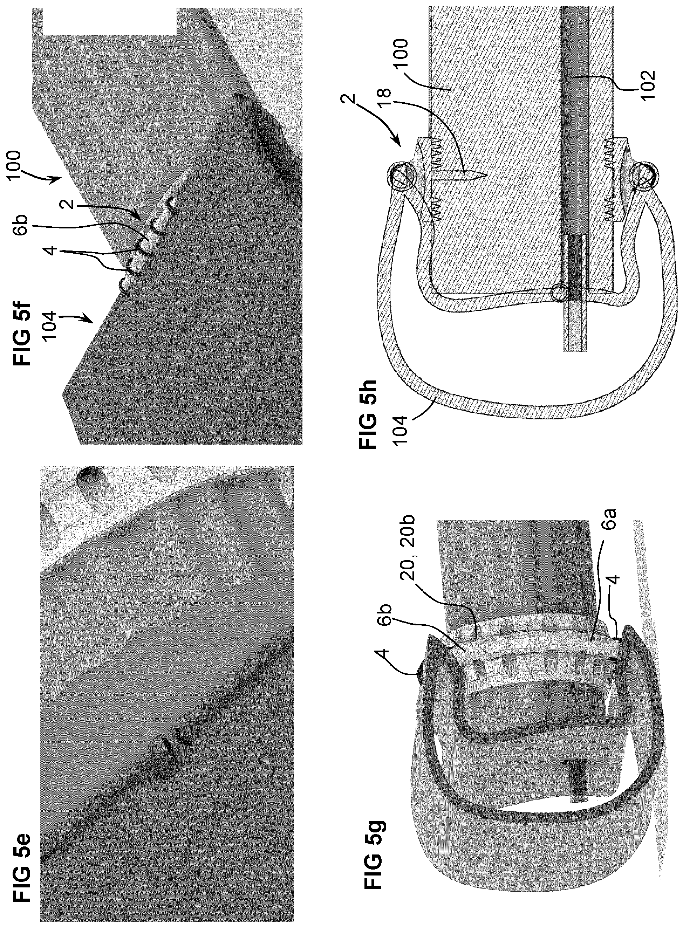

In the second embodiment illustrated in figures 4a to 4c and 5a to 5h, the upper ring portion is coupled to the lower ring portion by a pivot hinge 8c allowing the upper and lower ring portions 6a, 6b to be spread apart as illustrated in figures 4a and 4b and then closed together around the section of tissue 100 as illustrated for instance in figure 5b, optionally comprising a latching or locking element to hold the upper and lower ring portions together in the closed and locked position once assembled to the section of tissue.

The support ring 6 comprises spikes 18 that project into the central cavity 24, configured to engage the section of tissue received within the cavity to securely fasten the support ring thereto.

The support ring 6 according to various embodiments may further comprise suture guide holes 20, 20a, 20b for guiding and securing suture threads sewn by the surgeon through the suture holes and tissue to which the support ring 6 is coupled with the suture threads.

In the embodiment of figures 1a to 1e, the suture guide holes 20 are in-plane suture guide holes 20a that allow the surgeon to pass a suture thread through the suture guide holes in the plane of the support ring 6 from one side to the other side to further secure the first tissue section 100 received within the cavity 24 of the support ring in addition to the spikes 18.

It may be noted that in a variant of the embodiment of figures 1a to 1e, the suture guide holes 20a may be omitted in view of the fastening of the support ring to the first tissue section 100 with the spikes 18.

In the first embodiment of figures 1a to 1e, the support ring comprises a centre body portion 10 and one or two rims 12, 14 on the front and/or back side of the centre body portion 10. The centre body portion and rim or rims provide tie grooves 22, in particular a tie centre groove 22a and one or two tie lateral grooves in the rims 22b. The tie grooves serve to receive, position and secure tie threads used to secure the upper ring portion and lower ring portion 6a, 6b around the first tissue section 100 received within the support ring cavity 24, and further to locate and secure the tie thread that is fixed around a tubular second tissue section 104 that is received around the first tissue section 100 and support ring 6.

The support ring 6 may comprise inner peripheral teeth 16 that grip into the outer surface of the first tissue section received within the support ring cavity 24, to limit the axial movement of the first tissue section relative to the support ring.

In view of the spikes 18 that also provide the function of limiting the axial movement of the first tissue section 100 relative to the support ring 6, the inner peripheral grip teeth 16 are optional.

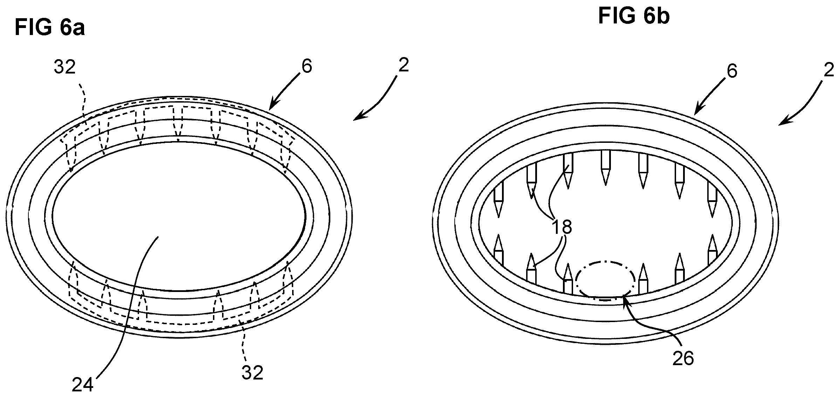

The support ring 6 comprises a spike free central zone 26 that does not comprise a spike, on at least one lower or upper side of the central cavity 24, configured to avoid piercing the pancreatic duct 102. In the embodiments with a two part support ring, at least one of the upper and lower ring portions 6a, 6b, or both ring portions, comprise(s) a spike free central zone 26. In the embodiment of figures 6a, 6b at least a bottom or a top side, or both the bottom and top sides comprise(s) a spike free central zone 26.

The tool kit 3 according to an embodiment of the invention serves to facilitate assembly and manipulation of the support ring 6 relative to the tissue sections to which it is coupled, and also provide means to guide the suture and tie thread procedures performed by the surgeon. The tool kit is in particular useful for the first embodiment, which may serve as a termino-terminal anastomosis ring configured for pancreatico-jejunal anastomosis, performed in open surgery.

The tool kit 3 may comprise a ring stabilization tool 5 that has a docking interface portion 21 adapted to engage the support ring 6, preferably, in an embodiment, in the tie centre groove 22a. The docking interface portion is configured to ensure that the upper and lower ring portions 6a, 6b are correctly positioned and completely engaged together in the closed ring position. The ring stabilization tool further comprises a grip portion 23 that can be gripped by the surgeon to hold and manipulate the support ring 6 prior to winding and fixing a tie thread in the tie lateral groove 22b to secure the two ring portions together.

The tool kit 3 may further comprise a holder fork 7 that has means to engage, in particular to clip, onto the support ring 6 in order to allow the surgeon to manipulate and position the support ring 6 and first tissue section 100 with respect to the second tubular tissue section 104 during the coupling procedure between the first and second tissue sections. The holder fork 7 comprises a pair of arms 9 spaced apart, the arms comprising a catch 11 at the ends thereof for removably engaging an outer portion of the support ring 6.

The holder fork comprises tool fixing elements 9, for instance in the form of locating orifices 15 or locating protruberances (not shown) receiving corresponding locating pin portions 25, 27 of a suture alignment tool 17 or a tie alignment tool 19 also forming part of the tool kit.

The suture alignment tool 17 comprises guide tubes 29 for receiving and guiding therethrough a suture needle 30 and suture thread 4 through the in-plane suture guide holes 20a.

The tie alignment tool 19 is configured to be positioned around the outer tubular second tissue section to locate the position of at least one of the tie grooves 22, for instance the tie centre groove 22a, such that a tie thread 4 can be positioned around the outer tissue section by the surgeon using guidance from the tie alignment tool 19.

The suture and tie alignment tools 17, 19, and the holder fork tool 7 may be removed from the implantable anastomosis device 2 when the first and second tissue sections 100, 104 are coupled together.

In the embodiment of figures 4a to 5h, suture guide holes 20 are provided as axial suture guide holes 22b that are distributed around an outer peripheral ridge 30 of the centre body portion 10 allowing the surgeon to anchor the suture threads 4 coupling the ring 6 to the second tissue section 104 in the lateral procedure illustrated in figures 5a to 5h.

It may be noted that in a variant of the first embodiment, instead of completely separate upper and lower ring portions, the upper and lower ring portions may be connected together prior to use with a hinge similar to the second embodiment.

The pivot hinge is illustrated as an assembled hinge mechanism, however other hinge devices may be implemented within the scope of the invention, for instance a hinge in the form of a flexible web interconnecting the upper and lower ring portions. The flexible web may for instance be integrally formed in an injection molding or 3D printing process with the upper and lower ring portions.

Referring to figures 6a to 6b, in a third embodiment, the support ring 6 may be provided as a closed single part support ring comprising spikes 18 mounted on a movable spike support 32. The support ring in this embodiment may be inserted over the inner first tissue section, and subsequently the spike supports 32 and spikes extending thereform are pushed inwardly. The spikes 18 may for instance be guided with corresponding orifices (not shown) in the support ring 6, Locking barbs or catches between the spike supports and ring support may engage to lock the spikes to the support ring in the fully engaged position as illustrated in figure 6b. The spike members 18 may be provided with latches, friction fit, or be secured in the final position by a suture tie thread.

In an aspect of the invention, the anastomosis device is adapted for Pancreaticoduodenectomy (also known as Whipple Procedure), for indications including pancreatic cancer, distal bile duct cancer and other pre-malignant conditions located in the head of the pancreas.

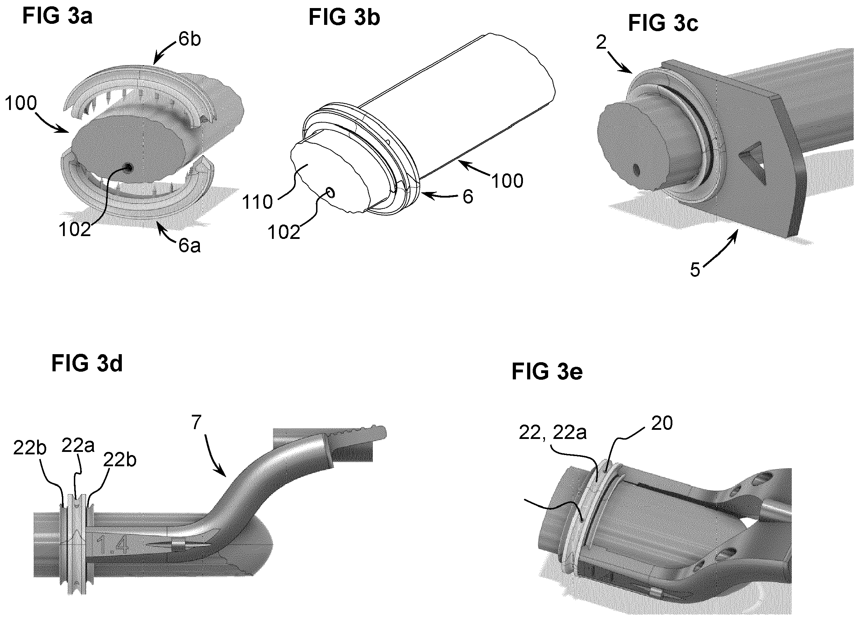

Referring to figures 3a to 3i, the implantable anastomosis device 2 according to a first embodiment is a termino-terminal anastomosis ring configured for pancreatico-jejunal anastomosis, performed in open surgery.

Referring to figures 3a to 3c, the lower portion 6a of the support ring 6 is positioned under the pancreatic stump 100 about 1.5cm from its medial face 110. The ring's upper portion 6b is closed on the lower portion 6a by gentle pressure, allowing the ring's spikes 18 to penetrate the pancreas. The two portions 6a, 6b of the support ring 6 are self-aligning due to the male-female coupling joint 8a, 8b. Once closed, the support ring 6 is temporarily stabilized using the ring stabilisation tool 5. Tie threads 4 are tightened around the tie lateral grooves 22b and the ring stabilisation tool 5 is removed.

Referring to figures 3d and 3e, the holder fork 7 is then clipped to the support ring 6. The pair of arms 9 and associated catches 11 are configured to clip to the support ring in a predefined and fixed rotational position.

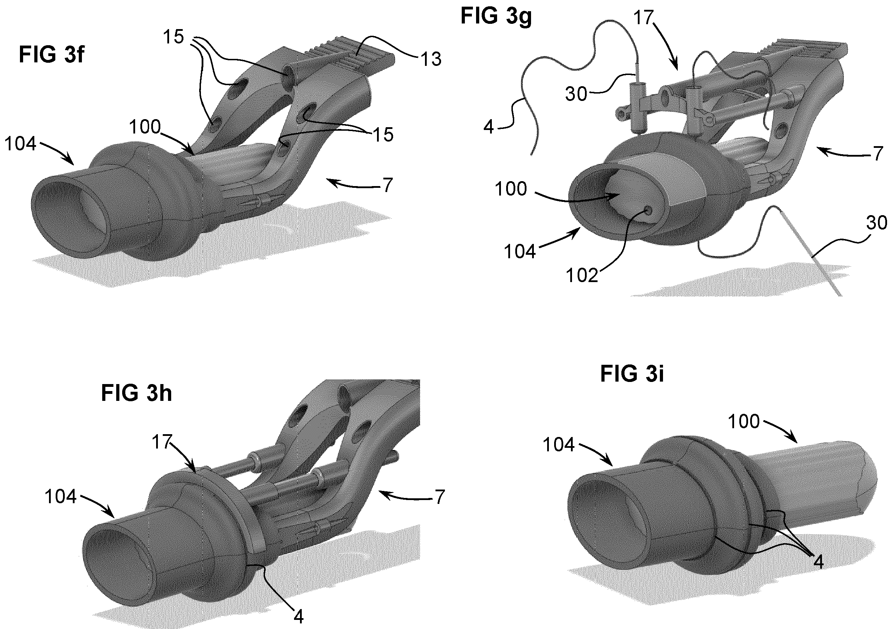

Referring to figures 3f and 3g, the support ring 6 and pancreas 100 are inserted into the open end of the jejunum 104 until the edge of the bowel overlaps the support ring 6 by about one centimeter.

A suture aligment tool 17 is plugged to the holder fork 7, whereby the locating pin portions 25 are inserted into the tool fixing orifices 15. Straight needle 30 sutures are passed through the suture guide tubes 29.

Referring to figures 3h and 3i, the suture alignment tool 17 is removed, and the sutures 4 are tightened around the support ring's central groove 22a. A tie alignment tool 19 is then plugged to the holder fork 7, whereby the locating pin portions 27 are inserted into the tool fixing orifices 15. A tie thread is secured around the central groove 22a of the support ring 6. One strong suture may be loosely tightened proximal to the support ring to seal the jejunal mucosa against the surface of the pancreas. The suture alignment tool 17 is removed, and an extra suture may be tightened distal to the support ring to increase water tightness.

Referring to figures 5a to 5h, the implantable anastomosis device 2 according to a second embodiment is a termino-lateral anastomosis ring configured for pancreatico-jejunal anastomosis, which may be suitable for robotic, laparoscopic, and open surgery.

Referring to figures 5a and 5b, this version of the implantable anastomosis device 2 is meant to reinforce the outer layer of any variant of the termino-lateral pancreatico jejunostomy. The support ring 6 upper and lower portions 6a, 6b are pivotally closed around the pancreatic stump 100 and locked in the fully closed position thanks to a clip mechanism.

Referring to figures 5c to 5e, the jejunum 104 is then stitched with suture thread 4 to the lower portion 6a of the support ring 6 using the axial suture guide holes 20b, forming the posterior wall of the anastomosis. A duct-to-mucosa 102, 108 anastomosis may then be constructed according to a *per se* standard technique.

Referring to figures 5f to 5h, then the anterior wall of the anastomosis is constructed by stitching with suture thread 4 the jejunum 104 on the upper portion 6b of the support ring 6 using the axial suture guide holes 20b.

### List of references

Medical anastomosis kit
**Implantable anastomosis device 2**
   in particular for Pancreaticoduodenectomy
**Support ring 6**
   Central cavity 24
   Upper ring portion 6a
   Lower ring portion 6b
      Coupling joint 8
      Complementary guide and positioning 8a, 8b
      Pivot hinge 8c
   **Centre body portion 10**
   **Rim(s) 12, 14**
      **Inner peripheral grip (teeth) 16**
   **Spikes 18**
      Spike free central zone 26
      Spike support 32
   **Suture guide holes 20**
      In-plane suture guide holes 20a (embodiment : Terminal anastomosis ring)
      Axial suture guide holes 20b (embodiment: Termino-Lateral anastomosis ring)
   **Tie grooves 22**
      Tie centre groove (in Centre body portion) 22a
      Tie lateral groove(s) (in rim(s)) 22b
Suture thread 4
**Tool kit 3**
   **Ring stabilisation tool 5**
      Docking interface 21
      Grip portion 23
   **Holder Fork 7**
      Arms (pair) 9
         Catch 11
      Finger grip 13
      Tool Fixing elements 15 (tool fixing orifices)
   **Suture alignment tool 17**
      Locating pin portions 25
      Suture guide tubes 29
   **Tie alignment tool 19**
      Locating pin portions 27
   Suture needle 30
   Pancreas 100 (pancreatic stump)
      Pancreatic duct 102
      Medial face 110
   Duodenum 104
      Orifice 108
   Jejunum 106

## Claims

1. Implantable anastomosis device (2) for use in a surgical connection between two tissues, comprising a support ring (6) having a central cavity (24) configured for receiving a first section of tissue therein, a plurality of spikes (18) integrally formed with or attached to the support ring, **characterized in that** the plurality of spikes are arranged to project into the central cavity, and the device further comprises a plurality of suture guide holes (20), or at least one peripheral tie groove (22), formed through or on the support ring and configured for securing a second section of tissue to the support ring with suture thread (4).

2. The device according to any preceding claim wherein at least one of a lower or an upper side of the central cavity (24) of the support ring comprises a spike free central zone (26) that does not comprise a spike.

3. The device according to any preceding claim wherein the support ring has an oval shape.

4. The device according to any preceding claim wherein the support ring is made of a non-absorbable polymer material, for instance nylon, or an absorbable polymer, for instance poly lactic-co-glycolic acid, and wherein the support ring is made by injection molding or 3D printing.

5. The device according to the claim 1 wherein the support ring comprises an upper portion (6b) and a lower portion (6a) movable with respect to each other from an open position configured to place the first section of tissue on one of the lower portion and upper portion, to a closed position in which the lower portion and upper portion form closed ring configured to fully surround the first section of tissue.

6. The device according to the preceding claim wherein the upper and lower portions prior to use are separate parts configured to be assembled together in use.

7. The device according to any preceding claim wherein the support ring comprises said at least one tie groove formed in a periphery of the support ring.

8. The device according to any preceding claim wherein the support ring comprises a plurality of tie grooves, one tie groove (22a) formed in a periphery of a central body portion (10) and at least another tie groove (22b) formed in a rim (12, 14) on a front side or a back side of the central body portion.

9. The device according to any preceding claim 1-7 wherein the support ring comprises said plurality of suture guide holes (20).

10. The device according to the preceding claim wherein the plurality of suture guide holes extend axially through an outer ridge (30) of the support ring (6).

11. The device according to any preceding claim configured for Pancreaticoduodenectomy.

12. The device according to the preceding claim in conjunction with any of claims 1-9 wherein the device is configured for termino-terminal pancreatico-jejunal anastomosis.

13. The device according to claim 11 in conjunction with claim 9 or 10 wherein the device is configured for termino-lateral pancreatico-jejunal anastomosis.

14. A tool kit for assembly and manipulation of the device according to any one of claims 1-9 and 12, including a holder fork (7) comprising a finger grip (13) and a pair of spaced apart arms (9) with catches (11) configured for removably coupling to an outer portion of the support ring.

15. The tool kit of the preceding claim further including any one or more of a suture alignment tool (17) comprising suture guide tubes (29) and a tie alignment tool (19), each comprising locating pin portions (25, 27) configured for engaging complementary positioning elements on the holder fork.

## Patentansprüche

1. Implantierbare Anastomosevorrichtung (2) zur Verwendung bei einer chirurgischen Verbindung zwischen zwei Geweben, die einen Stützring (6) mit einem zentralen Hohlraum (24), der dazu ausgelegt ist, einen ersten Bereich von Gewebe darin aufzunehmen, eine Vielzahl von Dornen (18), die integral im Stützring gebildet oder an demselben befestigt sind, umfasst, **dadurch gekennzeichnet, dass** die Vielzahl von Dornen angeordnet sind, in den zentralen Hohlraum vorzustehen, und die Vorrichtung ferner eine Vielzahl von Nahtführungslöchern (20) oder mindestens eine periphere Bindungsnut (22) umfasst, die durch den Stützring oder an demselben gebildet und zum Sicheren eines zweiten Bereichs von Gewebe mit einem Nahtfaden (4) am Stützring ausgelegt sind.

2. Vorrichtung nach einem der vorhergehenden Ansprüche, wobei mindestens eine einer unteren oder einer oberen Seite des zentralen Hohlraums (24) des Stützrings eine dornenfreie zentrale Zone (26) umfasst, die keinen Dorn umfasst.

3. Vorrichtung nach einem der vorhergehenden Ansprüche, wobei der Stützring eine ovale Form aufweist.

4. Vorrichtung nach einem der vorhergehenden Ansprüche, wobei der Stützring aus einem nicht absorbierbaren Polymermaterial, beispielsweise Nylon, oder einem absorbierbaren Polymer, beispielsweise Polylactid-co-glycolid, besteht und wobei der Stützring durch Spritzgießen oder durch 3D-Drucken hergestellt wird.

5. Vorrichtung nach Anspruch 1, wobei der Stützring einen oberen Abschnitt (6b) und einen unteren Abschnitt (6a) umfasst, die mit Bezug aufeinander aus einer offenen Position, die dazu ausgelegt ist, den ersten Bereich von Gewebe auf einem des unteren Abschnitts und des oberen Abschnitts zu platzieren, zu einer geschlossenen Position bewegbar sind, in der der untere Abschnitt und der obere Abschnitt einen geschlossenen Ring bilden, der dazu ausgelegt ist, den ersten Bereich von Gewebe vollständig zu umgeben.

6. Vorrichtung nach dem vorhergehenden Anspruch, wobei der obere und der untere Abschnitt vor der Verwendung separate Teile sind, die dazu ausgelegt sind, bei der Verwendung zusammengesetzt zu werden.

7. Vorrichtung nach einem der vorhergehenden Ansprüche, wobei der Stützring die mindestens eine Bindungsnut umfasst, die in einer Peripherie des Stützrings gebildet ist.

8. Vorrichtung nach einem der vorhergehenden Ansprüche, wobei der Stützring eine Vielzahl von Bindungsnuten umfasst, wobei eine Bindungsnut (22a) in einer Peripherie eines zentralen Körperabschnitts (10) gebildet ist und mindestens eine weitere Bindungsnut (22b) in einem Rand (12, 14) auf einer Vorderseite oder einer Rückseite des zentralen Körperabschnitts gebildet ist.

9. Vorrichtung nach einem der vorhergehenden Ansprüche 1 bis 7, wobei der Stützring die Vielzahl von Nahtführungslöchern (20) umfasst.

10. Vorrichtung nach dem vorhergehenden Anspruch, wobei sich die Vielzahl von Nahtführungslöchern axial durch eine äußere Erhöhung (30) des Stützrings (6) erstrecken.

11. Vorrichtung nach einem der vorhergehenden Ansprüche, die für eine Duodenopankreatektomie ausgelegt ist.

12. Vorrichtung nach dem vorhergehenden Anspruch in Verbindung mit einem der Ansprüche 1 bis 9, wobei die Vorrichtung für eine termino-terminale pankreatikojejunale Anastomose ausgelegt ist.

13. Vorrichtung nach Anspruch 11 in Verbindung mit Anspruch 9 oder 10, wobei die Vorrichtung für eine termino-laterale Pankreatiko-Jejunostomie ausgelegt ist.

14. Werkzeugsatz zur Zusammensetzung und Manipulation der Vorrichtung nach einem der Ansprüche 1 bis 9 und 12, der eine Haltegabel (7) beinhaltet, die einen Fingergriff (13) und ein Paar von beabstandeten Armen (9) mit Mitnehmern (11) umfasst, die zum entfernbaren Koppeln an einen Außenabschnitt des Stützrings ausgelegt ist.

15. Werkzeugsatz nach dem vorhergehenden Anspruch, der ferner eines oder mehreres von einem Nahtausrichtwerkzeug (17), das Nahtführungsrohre (29) umfasst, und einem Bindungsausrichtwerkzeug (19) beinhaltet, von denen jedes Lokalisierungsstiftabschnitte (25, 27) umfasst, die zum Eingreifen in komplementäre Positionierungselemente an der Haltegabel ausgelegt sind.

## Revendications

1. Dispositif d'anastomose implantable (2) destiné à être utilisé dans une connexion chirurgicale entre deux tissus, comprenant un anneau de support (6) ayant une cavité (24) centrale configurée pour recevoir une première section de tissu à l'intérieur, une pluralité de pointes (18) formées d'un seul tenant avec l'anneau de support ou fixées à celui-ci, **caractérisé en ce que** la pluralité de pointes sont agencées de manière à faire saillie dans la cavité centrale, et le dispositif comprend en outre une pluralité de trous de guidage de fil de suture (20), ou au moins une rainure de ligature périphérique (22) formée à travers l'anneau de support ou sur celui-ci et configurée pour solidariser une deuxième section de tissu et l'anneau de support à l'aide d'un fil de suture (4).

2. Dispositif selon l'une des revendications précédentes, dans lequel au moins un parmi un côté inférieur ou un côté supérieur de la cavité (24) centrale de l'anneau de support comprend une zone centrale exempte de pointes (26) qui ne comporte pas de pointes.

3. Dispositif selon l'une des revendications précédentes, dans lequel l'anneau de support est de forme ovale.

4. Dispositif selon l'une des revendications précédentes, dans lequel l'anneau de support est réalisé en un matériau polymère non résorbable, par exemple le nylon, ou un polymère résorbable, par exemple l'acide polylactique-co-glycolique, et dans lequel l'anneau de support est réalisé par moulage par injection ou par impression 3D.

5. Dispositif selon la revendication 1, dans lequel l'anneau de support comprend une partie supérieure (6b) et une partie inférieure (6a) mobiles l'une par rapport à l'autre pour passer d'une position ouverte configurée pour placer la première section de tissu sur la partie inférieure ou la partie supérieure, à une position fermée dans laquelle la partie inférieure et la partie supérieure forment un anneau fermé configuré pour entourer entièrement la première section de tissu.

6. Dispositif selon la revendication précédente, dans lequel, avant utilisation, les parties supérieure et inférieure sont des pièces séparées configurées pour être assemblées l'une à l'autre en cours d'utilisation.

7. Dispositif selon l'une des revendications précédentes, dans lequel l'anneau de support comprend ladite au moins une rainure de ligature formée sur la périphérie de l'anneau de support.

8. Dispositif selon l'une des revendications précédentes, dans lequel l'anneau de support comprend une pluralité de rainures de ligature, une rainure de ligature (22a) étant formée sur la périphérie d'une partie de corps centrale (10) et au moins une autre rainure de ligature (22b) étant formée dans un rebord (12, 14) sur un côté avant ou un côté arrière de la partie de corps centrale.

9. Dispositif selon l'une des revendications précédentes 1 à 7, dans lequel l'anneau de support comprend ladite pluralité de trous de guidage de fil de suture (20).

10. Dispositif selon la revendication précédente, dans lequel les trous de la pluralité de trous de guidage de fil de suture s'étendent axialement à travers une arête (30) extérieure de l'anneau de support (6).

11. Dispositif selon l'une des revendications précédentes configuré pour une pancréatoduodénectomie.

12. Dispositif selon la revendication précédente en conjonction avec l'une des revendications 1 à 9, dans lequel le dispositif est configuré pour une anastomose pancréatico-jéjunale termino-terminale.

13. Dispositif selon la revendication 11 en conjonction avec la revendication 9 ou 10, dans lequel le dispositif est configuré pour une anastomose pancréatico-jéjunale terminolatérale.

14. Trousse d'outils (3) destinée à l'assemblage et à la manipulation du dispositif selon l'une des revendications 1 à 9 et la revendication 12, comportant une fourche de maintien (7) comprenant une poignée pour les doigts (13) et une paire de bras (9) espacés munis de crans d'arrêt (11) configurés pour se coupler de manière amovible à une partie extérieure de l'anneau de support.

15. Trousse d'outils (3) selon la revendication précédente, comportant en outre un outil d'alignement de fil de suture (17) comprenant des tubes de guidage de fil de suture (29) et/ou un outil d'alignement de lien (19), chacun comprenant des parties de broche de mise en place (25, 27) configurées pour se mettre en prise avec des éléments de positionnement complémentaires sur la fourche de maintien.
